Europäisches Patentamt

European Patent Office

Office européen des brevets

(19)

(11) Publication number: **0 346 087**
**A2**

# EUROPEAN PATENT APPLICATION

(12)

(21) Application number: **89305730.7**

(22) Date of filing: **07.06.89**

(51) Int. Cl.⁴: **C 12 P 21/00**
**C 07 K 15/00**
**// A61K39/395, G01N33/577**

(30) Priority: **09.06.88 JP 141959/88**

(43) Date of publication of application:
**13.12.89 Bulletin 89/50**

(84) Designated Contracting States: **DE FR GB**

(71) Applicant: **SNOW BRAND MILK PRODUCTS CO., LTD.**
**6-1-1, Naebo-cho Higashi-ku**
**Sapporo-shi Hokkaido 065 (JP)**

(72) Inventor: **Shinmoto, Hiroshi**
**Sanraizu 109 1-3-61, Asahicho**
**Kawagoe-shi Saitama-ken (JP)**

**Kawasaki, Yoshihiro**
**Yukijirushidokushinryo 5-11-3, Arajukucho**
**Kawagoe-shi Saitama-ken (JP)**

**Dosaka, Shinichi**
**5-15-39-616, Kitaurawa**
**Urawa-shi Saitama-ken (JP)**

**Murakami, Hiroki**
**4-16-16, Najima Higashi-ku**
**Fukuoka-shi Fukuoka-ken (JP)**

(74) Representative: **West, Alan Harry et al**
**R.G.C. Jenkins & Co. 26 Caxton Street**
**London SW1H 0RJ (GB)**

(54) Hybrid antibody and process for the production thereof.

(57) A polymer-type hybrid antibody molecule having the capability to recognize two different antigens and a process for the production thereof are disclosed, wherein two kinds of antibody molecules, each having different antigen specificity, are polymerized by cross-linkage of carboxyl groups of carboxyl ends thereof via amino groups. The resultant hybrid antibody can be used, for example, in microdetermination or as a therapeutic agent.

EP 0 346 087 A2

**Description**

## HYBRID ANTIBODY AND PROCESS FOR THE PRODUCTION THEREOF

The invention relates to a so-called bifunctional antibody (hereinafter referred to as bf antibody) which is capable of recognizing two different antigens and to a process for the production thereof.

It is possible to use bf antibody as a therapeutic agent for cancer, infectious diseases or the like and as a diagnostic agent for various diseases and in microdetermination.

An antigen-antibody interaction known as the most typical immunological reaction that occurs in the body has been widely used in therapy of various diseases such as cancer, infectious diseases and intoxications, biochemical blood tests and immunological diagnosis or microdetermination because of the high specificity of antibody in the interaction. It is worth mentioning that a process for producing monoclonal antibodies using a hybridoma method published by Milsteins et al. (Nature, 256, 495-497 (1975)) provided a technique of producing only one kind of antibody in large quantities by a relatively simple process. Owing to this technique, keen attention has been arrested to the possible use of antibodies.

Furthermore, by the advantageous use of the high specificity of antibody, various methods has been under investigation to enable one antibody molecule to recognize two different antigens so that the field of the utilization of the antibody can be enlarged. In the course of the investigation, methods have been suggested, in which two kinds of antibodies, each having different antigen recognition specificity, are linked together to obtain one antibody molecule.

Suggested methods of binding two kinds of antibodies include (1) chemical binding; (2) binding via protein A produced by Staphylococcus aureus and (3) binding by culturing cells of a fusion cell line in which two kinds of antibody-producing cells had been fused so as to obtain a bound antibody complex from the culture.

Among the above-mentioned methods, techniques for chemical binding have been disclosed in a method by Nisonoff et al. (A. Nisonoff et al., Arch. Biochem. Biophys, 90, 460-462 (1961)) in which after antibodies are treated with pepsin, disulfide cross-linkages between the heavy chains of antibodies are reduced by mercaptoethylamine and then reoxidized to obtain bf antibodies, or in a method in which cross-linkage reactions are carried out using glutaric dialdehyde or N-succinimidyl 3-(2-pyridyldithio) propionate (Y. Germani et al., J. Immun. Methods, 98, 83-89 (1978)). Furthermore, a method in which an F(ab')2-fragment of a monoclonal antibody is transformed into a thionitrobenzoic acid derivative and then allowed to react with a reduced product of another Fab'-fragment to obtain a bf antibody molecule (M. Bermani et al., Science, 229, 81-83 (1985)).

However, in the antibodies thus prepared by binding antibody molecules via disulfide groups, the antigen binding sites of the respective antibodies are located closely one another. Accordingly, it is pointed out that, in some cases, when one antigen is bound to one of the binding sites of the integrated antibody, binding of another antigen to be bound to the other binding site may be interfered due to the resultant three-dimensional obstacles.

Furthermore, in the above-mentioned cross-linkage method using glutaric dialdehyde or N-succinimidyl 3-(2-pyridyldithio) propionate, antibodies are linked together via amino groups. In antibodies, antigen binding sites are located at amino terminals. Incidentally, it is possible that the antibodies are bound via these terminal amino groups. Thus, the resultant loss of antibody activity has been pointed out as a disadvantage of this binding method.

Further, in the method in which antibodies are bound via protein A, there is a disadvantage that a desired bf antibody may not be prepared in the case with IgA and IgM because antibodies of these classes are not capable of binding the protein A.

Furthermore, recently, a method of obtaining bf antibody by culturing cells of a fusion cell line has been disclosed by Milstein et al. (Nature, 305, 537-540 (1987)).

However, this method entails the complicated procedure to obtain the cell line for the production of a desired bf antibody and, furthermore, productivity of the obtained cell lines was not always stable. Particularly, some cell lines lost capability of bf antibody production, and the amounts of bf antibodies produced were gradually declined in some cell lines. Accordingly, a careful attention is always required in the bf antibody production by the cell lines and also in the maintenance of the cell lines.

Under these circumstances, a novel method for the production of bf antibody, which is applicable for the production of a wide range of antibody classes, and the production of novel bf antibodies have been anticipated.

One aspect of the present invention is to provide a new type hybrid antibody molecule having a polymerization structure in which two kinds of antibody molecules are bound via cross-linkages at the respective carboxyl groups of carboxyl ends and the method for the production thereof. Namely, according to the present invention, a new type, namely polymer type, of bf antibody which is different from conventional bf antibodies and have polymerization structure via carboxyl groups at carboxyl ends.

Furthermore, any classes of antibodies such as IgM which are difficult to prepare in the conventional manner can be easily constructed into bf antibodies, which enlarges the field of the utilization of antibodies.

A hybrid antibody (bf antibody) according to the present invention is a novel polymer-type bf antibody which has a polymerization structure in which two different antibody molecules are bound by means of cross-linking between the carboxyl groups of carboxyl ends of individual molecules via amino groups such as diamines, has the binding activity to the two antigens, and furthermore has physicochemical features as a single molecule antibody.

A bf antibody of the present invention can be prepared in the procedure described hereinafter.

Antibodies to be used in the present invention can be selected from any kinds of monomer-type antibodies different from one another. IgG-class antibodies are most generally used but antibodies of IgA, IgM and other classes can also be used.

Furthermore, antibodies of any species can be used. Both polyclonal and monoclonal antibodies can be use.

Examples of the antibodies include polyclonal antibodies of human, chimpanzee, goat, sheep, pig, horse, rabbit, guinea pig, rat, mouse or the like and monoclonal antibodies of human, rat, mouse or the like.

An equal amount of two kinds of antibodies having been selected from the above-mentioned examples are dissolved in an appropriate buffer solution (pH 4 to 10) such as phosphate buffer, and a condensing agent is added on ice to the solution in an amount of 10- to 1,000-fold molarity of the total of the two antibodies. Then, immediately, diamine in an amount of 2- to 4-fold molarity of the condensing agent was added and cross-linkage reaction is carried out at room temperature for 1 to 24 hours. After the reaction, low molecular-weight diamine and the condensing agent are removed preferably by subjecting the reaction mixture to gel filtration on a column or ultrafiltration, so that bf antibodies produced by cross-linkage polymerization are obtained.

Examples of the condensing agent to be used in the reaction include water-soluble carbodiimide compounds such as 1-ethyl-3(3-dimetylaminopropyl) carbodiimide (EDC), 1-cyclohexyl-3-(3-morpholinoethyl) carbodiimide methoparatoluene sulfonate (CMC), 1-benzyl-3-(3-dimethylaminopropyl) carbodiimide (BDC) and 1-cyclohexyl-3(morpholinoethyl) carbodiimide, and the salts thereof. Further, other carbodiimide compounds can be used only in an organic solvent.

Water-soluble diamines of very different kinds, such as 1,3-diaminopropane, 1,4-diaminobutane, 1,7-diaminoheptane and 1,8-diaminooctane, can be used. Water-soluble diamines having relatively long carbon chains between amines, such as 1,5-diaminopentane, 1,6-diaminohexane and salts thereof with acids, are preferably used.

According to the present invention, a desired bf antibody is prepared by condensation reaction of respective carboxyl groups of two kinds of antibody molecules with diamine used as a cross-linking agent. In consequence, the resultant bf antibody is a polymer-type bf antibody in which diamine is covalently bound as a spacer.

A process for the production of bf antibody according to the present invention is simple as compared to the conventional chemical method for the production of monomer-type bf antibodies, has an advantage that the bf antibody can be prepared from antibodies of all the classes, and furthermore, does not require complicated steps which is essential to the cell fusion method.

The production of the bf antibody obtained according to the present invention can be confirmed by determining the antigen specificity of immunoglobulin class and subclass used for the production.

Namely, the production of the bf antibody can be confirmed by detecting that the resultant antibody has specificity to two kinds of antigens, namely two kinds of immunoglobulin classes or subclasses in one molecule, using agar-gel double diffusion method, erythrocyte aggultination reaction, latex aggultination reaction, enzyme immunoassay, radioimmunoassay, blotting analysis or the like.

The following Examples serve to illustrate the present invention in detail.

Example 1:

Preparation of bf antibodies from mouse monoclonal antibodies and human monoclonal antibodies

In 1 ml of 0.7 M phosphate buffer (pH 7), 1.5 mg (10 nanomoles) each of mouse anti-bovine lactoferrin monoclonal antibodies (IgG class) (Kawakami et al., J. Dairy sci., 70, 752 (1987) and human anti-influenza A-type virus monoclonal antibodies (IgG class) (Japanese Patent Laid-Open Pub. No. 51700/1987) was dissolved, and then the solution was taken into a small glass test tube and cooled on ice.

To the solution, 1.92 mg (10 μM) of EDC chloride was added as a condensing agent, immediately followed by addition of 4.73 mg (25 μM) of 1,6-diaminohexane chloride, and the mixture was thoroughly mixed and allowed to react for 6 hours at room temperature,

After the reaction, the reaction mixture was subjected to gel filtration using a Sephadex G-50 column (1 x 20 cm, Pharmacia) which had been equilibrated with phosphate buffered saline (PBS) (pH 7.2), and the voided fraction was then collected. The amount of the antibody preparation obtained was 2.8 mg. The antibody preparation was subjected to gel filtration using a Superose 6 column (Pharmacia) for fractionation, which revealed that the molecular weight of the obtained antibodies ranged from 150,000 to 3,000,000.

Further, the antibodies obtained in the above-mentioned manner were confirmed to be bf antibodies by an enzyme-linked immunosorbent assay (ELISA) as follows. Confirmation of bf antibodies by ELISA:

Anti-mouse IgG and anti-human IgG (Tago) were respectively diluted 1,000 times with 0.05 M sodium bicarbonate, and 50 microliters of the diluted solutions was dispensed into each of wells of an ELISA plate (NUNC immunoplate II, Inter Met). The plate was then allowed to stand at room temperature overnight. Then, 50 microliters of a 4-fold diluted solution of the antibodies (the fraction obtained by the gel filtration) was added into each of the wells and the plate was allowed to stand at room temperature for 1 hour. The plate was washed three times with a 10-fold diluted Block Ace solution (Dainippon Pharmaceutical Co., Ltd.) containing 0.05 % Tween 20. 50 microliters each of 1,000-fold diluted solutions of anti-mouse IgG and anti-human IgG (Tago), both of which had been labeled with horseradish peroxidase, was respectively added into each well of the corresponding IgG, and the plate was allowed to stand at room temperature for 1 hour. The plate was washed six times, and then 100 microliters of 2,2'-azino-di-(3-ethylbenzylthiazolylsulphonate (ABTS) was added into each of the wells as a substrate so as to determine the enzyme activity bound to the plate.

Results are shown in Table 1. As evident from the results, the obtained antibodies were adsorbed to the wells coated with anti-mouse IgG and anti-human IgG, and all the adsorbed antibodies reacted with peroxidase-labeled anti-mouse IgG and anti-human IgG.

Thus, the obtained antibodies were confirmed to be bf antibodies comprising mouse IgG and human IgG.

Table 1

| Coated antigen | Enzyme-labeled antigen | Reactivity |
|---|---|---|
| Anti-mouse IgG | Anti-mouse IgG | + |
| Anti-mouse IgG | Anti-human IgG | + |
| Anti-human IgG | Anti-mouse IgG | + |
| Anti-human IgG | Anti-human IgG | + |

Example 2:

Preparation of bf antibodies from rabbit polyclonal antibodies

Commercially available rabbit anti-mouse IgG antibodies (IgG fraction, MBL) and rabbit anti-horseradish peroxidase antibodies (IgG fraction, MBL) were mixed, and the cross-linkage reaction was carried out in the same manner as described in Example 1, except that 1,5-diaminopentane chloride was used as a cross-linking agent.

The amount of antibodies produced from 1.5 mg each of the above-mentioned antibodies was 2.5 mg, and the average molecular weights of the antibodies produced ranged from 150,000 to 3,000,000. The produced antibodies were confirmed to be bf antibodies by analyzing antigen specificity in a manner as follows.

Confirmation of bf antibodies by antigen specificity:

Mouse serum IgG (Seikagaku Kogyo Co., Ltd.) was dissolved in 0.05 M NaHCO$_3$ at a concentration of 5 micrograms/ml. and 50 microliters of this solution was dispensed into each of wells of an ELISA plate for antigen coating. After blocking, 50 microliters of a diluted solution of the produced antibodies to be tested (100 ng/ml) and 20 microliters of peroxidase (1 microgram/ml, Sigma) were added into each of the wells, and the plate was allowed to stand for 1 hour. Then, after washing the plate, ABTS was added for color development.

Results are shown in Table 2.

Table 2

| Plate coating | Addition of antibodies to be tested | Peroxidase reaction |
|---|---|---|
| None | - | - |
| | + | - |
| Mouse serum IgG | - | - |
| | + | + |

As evidently shown in Table 2, peroxidase was bound to the plate only when the produced antibodies were allowed to react with the mouse serum IgG coated on the plate. Thus, the produced antibodies were confirmed to be bf antibodies.

Example 3:

Western blot analysis with bf antibodies

Western blot analysis of human IgA alpha-chain was carried out using the bf antibody, namely the anti-mouse IgG/anti-peroxidase antibodies prepared in Example 2, in a manner as described below.

Western blot analysis:

Pretreatment was carried out by adding 50 microliters of 0.1 M tris-HCl buffer (pH 6.8) containing 2 % sodium dodesyl sulfate (SDS) and 5 % 2-mercapto ethanol to 50 microliters of human serum, followed by incubation at 37°C for 16 hours.

A sample of 5 microliters of the serum treated as described above was subjected to electrophoresis on a 10 % polyacrylamide gel according to the method of Laemmli (Nature, 277, 680 (1970)).

After the electrophoresis, the proteins fractionated in the polyacrylamide gel were electrically blotted onto PVDF transfer membrane filter (Daiichi Pure Chemicals Co., Ltd.) using an electric blotting device (Tafco). The filter was subjected to blocking using Block Ace for 1 hour and then allowed to react with 5 ml of a 10,000-fold diluted solution of mouse monoclonal anti-human IgA antibody (AMD, Nippon Kayaku) for 1 hour.

The filter was washed with Tween-PBS and then allowed to react with 5 ml of a diluted solution of the obtained bf antibodies (1 microgram/ml) for 30 minutes. The filter was washed again and allowed to react with 5 ml of a peroxidase solution (1 microgram/ml) for 30 minutes. Finally, peroxidase bound to the filter was located by developing color using 3,3'-diaminobenzidine (DAB) as a substrate. As a result, a band at a position of a molecular weight of about 50,000, corresponding to that of the alpha-chain molecule, was detected.

**Claims**

1. A polymer-type hybrid antibody molecule having the capability to recognize two different antigens, characterized in that two kinds of antibodies recognizing different antigens are polymerized by cross-linkage structures with binding of carboxyl groups at carboxyl ends of the respective antibody molecules.

2. A polymer-type hybrid antibody molecule as set forth in claim 1, in which said cross-linkage structures are polymerizing structure between amino groups of a diamine compound and carboxyl groups of the respective antibody molecules.

3. A process for the production of a polymer-type hybrid antibody molecule, which comprises allowing two kinds of antibodies recognizing different antigens to react with a condensing agent and a diamine compound for cross-linkage reaction and then removing low-molecular weight diamine and the condensing agent.

4. A process as set forth in claim 3, in which said low-molecular weight diamine and the condensing agent were removed by means of gel filtration or ultrafiltration.

5. A process as set forth in claim 3 or 4, in which said condensing agent is a water soluble carbodiimide.

6. A process as set forth in claim 3, in which said diamine is 1,5-diaminopentane or 1,6-diaminohexane.

7. A polymer-type hybrid antibody molecule as set forth in claim 1, in which said two kinds of antibody molecules are those of IgG class.

8. A polymer-type hybrid antibody molecule as set forth in claim 1, in which said two kinds of antibody molecules are those of immunoglobulin classes different one another.

9. A process as set forth in claim 3, in which said condensing agent is added in a molarity 10 - 1,000 times the total molarity of said two antibodies and said diamine is added in a molarity 2 - 4 times that of said condensing agent.